# EUROPÄISCHE PATENTSCHRIFT

(11) **EP 2 326 611 B1**
(45) Veröffentlichungstag und Bekanntmachung des Hinweises auf die Patenterteilung: **29.08.2012**
(21) Anmeldenummer: 09778255.1
(22) Anmeldetag: 02.09.2009
(51) Int. Cl.: C07C 17/156, C07C 19/045, B01J 8/00, B01J 8/18

(54) **VERFAHREN ZUR NUTZUNG DER IM HERSTELLUNGSPROZESS VON 1,2-DICHLORETHAN AUS ETHYLEN IN EINEM WIRBELSCHICHTREAKTOR ANFALLENDEN REAKTIONSWÄRME**
METHOD FOR USING THE REACTION HEAT DEVELOPING DURING THE PRODUCTION PROCESS OF 1,2-DICHLOROETHANE FROM ETHYLENE IN A FLUIDIZED BED REACTOR
PROCÉDÉ D'UTILISATION DE LA CHALEUR DE RÉACTION PRODUITE DANS UN RÉACTEUR À LIT FLUIDISÉ LORS DU PROCESSUS DE PRODUCTION DE 1,2-DICHLOROÉTHANE À PARTIR D'ÉTHYLÈNE

(30) Priorität: 23.09.2008 DE 102008048526
(43) Veröffentlichungstag der Anmeldung: 01.06.2011
(73) Patentinhaber: ThyssenKrupp Uhde GmbH, 44141 Dortmund (DE); Vinnolit Gmbh&Co. Kg, 84508 Burgkirchen (DE)
(72) Erfinder: GNABS, Ulrike, 65779 Kelkheim/Taunus (DE); BENJE, Michael, 65812 Bad Soden (DE); KERN, Walter, 65779 Kelkheim/Taunus (DE)
(74) Vertreter: Dabringhaus, Walter
(86) Internationale Anmeldenummer: PCT/EP2009/006329
(87) Internationale Veröffentlichungsnummer: WO 2010/034392

(56) Entgegenhaltungen:
- EP-A- 0 997 187
- DE-A1- 4 131 446
- DE-A1- 19 718 871
- DE-A1-102006 049 546
- US-A- 4 368 173

## Beschreibung

Die Erfindung richtet sich auf ein Verfahren zur Nutzung der bei der Herstellung von 1,2-Dichlorethan (EDC) aus Ethylen durch Umsetzung mit Sauerstoff und Chlorwasserstoff (Oxichlorierung) in einem Wirbelschichtreaktor entstehenden Reaktionswärme, mit Abführung dieser Reaktionswärme durch innerhalb des Reaktors befindliche in der Wirbelschicht positionierte Kühlrohrbündel.

Wirbelschichtreaktoren zur Oxichlorierung von Ethylen werden durch in die Wirbelschicht eingetauchte Kühlrohrbündel gekühlt (DE 197 18 871 A). In diesen Kühlrohrbündeln wird im Kreislauf geführtes Kesselspeisewasser verdampft. Dieser Dampf wird entweder zur Anlagengrenze abgegeben oder im Anlagenverbund des EDC/VCM-Prozesses zur Beheizung von Kolonnen oder Aufheizern verwendet. Hierbei ist man bestrebt, möglichst hochwertigen, d.h. heißen Dampf zu erzeugen, da dieser an verschiedenen Stellen des EDC/VCM-Prozesses zu Beheizungszwecken (z.B. Beheizung der VCM-Kolonne) benötigt wird. Ein radial durchströmter Festbettreaktor zur Durchführung heterogen katalytischer Gasphasenreaktionen, d.h. ein anderer Reaktortyp, ist aus der DE 41 31 446 A1 bekannt. US4368173 offenbart einen Prozess zur Oxichlorierung von Ethen in einem Wirbelschichtreaktor, welcher zur Abführung der Reaktionswärme in der Wirbelschicht positionierte Kühlrohrbündel enthält. Die Prozesswärme wird zur Dampferzeugung verwendet.

Die Baugröße eines Oxichlorierungsreaktors wird einerseits durch die erforderliche Kühlfläche zur Abführung der Reaktionswärme und andererseits durch die für eine bestimmte EDC-Produktionsmenge erforderliche Katalysatormenge und, dadurch bedingt, das Volumen der Wirbelschicht im fluidisierten Zustand bestimmt. Bedingt durch die aus konstruktiven Gründen erforderliche Baugröße des Kühlrohrbündels ist das tatsächliche Katalysatorinventar des Reaktors immer größer als für eine bestimmte gewünschte Produktionsmenge eigentlich erforderlich wäre bzw. die tatsächlich mit dem Katalysator erzielbare Raum-Zeit-Ausbeute wird aus konstruktiven Gründen nicht realisiert.

Zur Verkleinerung des Reaktors bzw. Erhöhung der Produktionsmenge bei gegebenem Reaktorvolumen, d.h. auch für eine bessere Ausnutzung des Katalysators (Prozessintensivierung) können prinzipiell zwei Wege beschritten werden:

### 1) Erhöhung der Reaktionstemperatur

Die Erhöhung der Reaktionstemperatur führt einerseits zu einer Erhöhung der Produktionsmenge (Raum-Zeit-Ausbeute) und andererseits (über eine Erhöhung der wirksamen Temperaturdifferenz für die Wärmeübertragung von der Wirbelschicht an das Kühlrohrbündel) zu einer verbesserten Wärmeabfuhr.

Nachteilig ist hierbei, dass bei einer Erhöhung der Reaktionstemperatur auch der Produktionsverlust durch Nebenreaktion, wie die Bildung von höher chlorierten Nebenprodukten oder die Oxidation von Ethylen zu CO und CO₂ (der Fachmann spricht von Ethylenabbrand) stark zunimmt und die Wirtschaftlichkeit des Verfahrens beeinträchtigt.

### 2) Erhöhung der wirksamen Temperaturdifferenz

Durch eine Erhöhung der wirksamen Temperaturdifferenz lassen sich die erforderliche Kühlfläche und damit die Baugröße des Kühlrohrbündels erheblich verringern, was auch zu einer Verkleinerung des Reaktors führt. Die Erhöhung der Temperaturdifferenz kann durch eine Erniedrigung des Druckes des erzeugten Dampfes erreicht werden. Dies führt jedoch dazu, dass eine große Menge niederwertigen Dampfes erzeugt wird, was wiederum die Wirtschaftlichkeit des Verfahrens verschlechtert.

Ebenso lässt sich die wirksame Temperaturdifferenz erhöhen, indem das Kesselspeisewasser vor Eintritt in das Kühlrohrbündel unterkühlt wird.

Der Wärmeübergangskoeffizient α, ₐᵤₛₛₑₙ (Wärmeübergang von der Wirbelschicht zur äußeren Kühlrohrwand) ist wesentlich geringer als der Wärmeübergangskoeffizient α, ᵢₙₙₑₙ (Wärmeübergang von der inneren Kühlrohrwand zum Kühlwasser) und zwar sowohl bei rein konvektivem Wärmeübergang als auch beim Verdampfen an der Innenseite des Kühlrohrs. Daher ändert sich der Gesamt-Wärmeübergangskoeffizient K beim teilweisen oder vollständigen Übergang von der Verdampfungs- zur konvektiven Kühlung nur wenig. Deutlich größer ist der Einfluss der wirksamen Temperaturdifferenz auf die pro Flächeneinheit übertragbare Wärmemenge.

Die Unterkühlung des Kesselspeisewassers kann auf verschiedene Arten erfolgen; so kann die Unterkühlung in einem Wärmeaustauscher mittels Luft- oder Wasserkühlung erfolgen. Diese Variante ist jedoch unwirtschaftlich, da die Wärme nicht mehr genutzt werden kann.

Eine weitere Variante ist die Abkühlung des Kesselspeisewassers durch Zwischenentspannung. Hier wird das Kesselspeisewasser in einem (zusätzlichen) Ausdampfbehälter auf einen niedrigeren Druck entspannt, wobei eine festgelegte Dampfmenge mit einer korrespondierenden Temperatur entsteht. Auch dieses Verfahren ist wirtschaftlich nachteilig, da niederwertiger Dampf erzeugt wird. Weiterhin wird zusätzliche Ausrüstung benötigt und der elektrische Energieverbrauch der Kesselspeisewasserpumpe steigt an, da das Kesselspeisewasser nach der Entspannung wieder auf den Ausgangsdruck gebracht werden muss.

Um den obigen Problemen gerecht zu werden, besteht die Aufgabe der Erfindung darin, die Nutzung der Wärme bei gleichzeitiger Verkleinerung der entsprechenden Anlagenelemente zu verbessern.

Mit einem Verfahren der eingangs bezeichneten Art wird diese Aufgabe gemäß der Erfindung dadurch gelöst, dass ein Teil der Reaktionswärme durch Erwärmung von Kesselspeisewasser abgeführt wird, wobei das erwärmte Kesselspeisewasser zur Beheizung von Wärmesenken im Herstellungsprozess von EDC, VCM, PVC oder in anderen Wärmesenken eingesetzt wird.

Der Vorteil der erfindungsgemäßen Verfahrensweise besteht darin, dass Wärmesenken im EDC/VCM/PVC-Anlageverbund nicht mehr, wie bisher, mit Dampf beheizt werden, sondern unmittelbar mit erhitztem Kesselspeisewasser.

Hierdurch kühlt sich das Kesselspeisewasser ab und kann dann zur neuerlichen Erwärmung wieder in die Kühlrohrbündel des Oxichlorierungsreaktors eingespeist werden. Dadurch wird die Reaktionswärme der Oxichlorierung weiterhin genutzt und die Wirtschaftlichkeit des Verfahrens bleibt erhalten. Es ist dabei möglich die Reaktionswärme vollständig durch Erwärmung von Kesselspeisewasser abzuführen oder aber das Kesselspeisewasser teilweise verdampfen zu lassen.

Ein besonderer Vorteil besteht darin, dass der Verschleiss auf der Kühlwasserseite bei entsprechend reduzierter Dampfmenge mit einhergehender reduzierter Strömungsgeschwindigkeit signifikant herabgesetzt wird.

Beispiele für geeignete Wärmesenken im Anlagenverbund der EDC/VCM/PVC-Herstellung sind:

| | |
|---|---|
| Umlaufverdampfer | VCM-Kolonne |
| Umlaufverdampfer | HCI-Kolonne |
| Umlaufverdampfer | VCM-Stripper |
| Umlaufverdampfer | Leichtsieder-Kolonne |
| Umlaufverdampfer | Entwässerungs-Kolonne |
| Vorwärmer | EDC (EDC-Spaltung) |
| Vorwärmer | Kreisgas (Oxichlorierung) |
| Vorwärmer | HCI (Oxichlorierung) |
| Trockner | (PVC-Trocknung) |

Die Erfindung ist jedoch nicht auf diese Beispiele beschränkt.

Weitere Ausgestaltungen, Merkmale und Vorteile ergeben sich aus den Unteransprüchen. Dabei kann vorgesehen sein, dass durch das durch die Reaktionswärme erwärmte Kesselspeisewasser Destillationskolonnen im Anlageverbund der Herstellung von 1,2-Dichlorethan/Vinylchlorid beheizt werden.

Eine andere Möglichkeit besteht erfindungsgemäß darin, dass durch das erwärmte Kesselspeisewasser Wärmetauscher zur Erwärmung von Prozessströmen im Anlagenverbund der Herstellung von 1,2-Dichlorethan/Vinylchlorid beheizt werden, wobei nach der Erfindung auch vorgesehen sein kann, dass durch das erwärmte Kesselspeisewasser eine Trocknungsvorrichtung von Polyvinylchlorid (PVC) beheizt wird.

Wie oben schon erwähnt können auch weitere Wärmesenken entsprechend aufgeheizt werden.

### Beispiele:

In einem Oxichlorierungsreaktor soll eine Wärmeleistung von 19221 kW übertragen werden. Die Wärmeübertragung erfolgt mittels in die Wirbelschicht eingetauchter Kühlrohrbündel von je 12 Rohren á 11,5 m Länge mit einem Außendurchmesser von 88,9 mm.

(Bei der Flächenberechnung wird nur die gerade Rohrlänge berücksichtigt; die verbindenden Rohrbögen werden vernachlässigt).

### 1 Wärmeübertragung durch Verdampfung

Es wird Kesselspeisewasser von 186° C verdampft (Dampfdruck ca. 11,5 bar abs.) Als Gesamt-Wärmeübertragungskoeffizient wurden 400 W/m² K bestimmt. Die Reaktionstemperatur (Temperatur der Wirbelschicht) beträgt 215° C. Mit diesen Daten lässt sich aus der erforderlichen Übertragungsleistung von 19221 kW eine erforderliche Wärmeaustauschfläche von 1657 m² berechnen, was ca 43 Kühlrohrbündeln entspricht. Je Kühlrohrbündel wird dabei eine Wärmeleistung von ca. 447 kW übertragen. Bei einer Verdampfungsenthalpie des Kesselspeisewassers von 1992,5 kJ/kg werden ca. 34,7 t Dampf/h erzeugt. Daraus ergibt sich bei einer Verdampfungsrate von 8 % eine Umwälzmenge von ca. 434 t/h Kesselspeisewasser.

### 2 Wärmeübertragung durch Erzeugung von Heißwasser

Die Wärmeleistung von 19221 kW/m² soll überwiegend durch Erwärmung von Kesselspeisewasser übertragen werden. Als Gesamt-Wärmeübertragungskoeffizient wurden 395 W/m² K bestimmt. Mittels einer nummerischen Berechnungsmethode wird iterativ die erforderliche Umwälzmenge an Kesselspeisewasser bestimmt, die sich auf der zur Verfügung stehenden Rohrlänge auf 186° C aufwärmt. Mit der gleichen Methode wird die dabei übertragene Wärmeleistung pro Kühlrohrbündel bestimmt. Es ergibt sich eine Kesselspeisewassermenge von 10,093 t/h pro Kühlrohrbündel. Durch die Aufwärmung des Kesselspeisewassers wird eine Wärmeleistung von ca. 320 kW/Kühlrohrbündel übertragen. Als erforderliche Rohrlänge für die Aufwärmung des Kesselspeisewassers ergeben sich ca. 70 m. Es stehen dann noch 138 - 70 = 68 m zur Verfügung, die einer Wärmeaustauschfläche von 19 m² entsprechen.

Mit dem Wärmeübergangskoeffizienten von 400 W/m² und der Temperaturdifferenz von 29° C (215 - 186) lässt sich eine durch Verdampfung übertragene Wärmeleistung von 220 kW berechnen. Insgesamt können also 320 + 220 = 540 kW übertragen werden, was einer Steigerung von ca. 20 % entspricht. Wird die Eintrittstemperatur des Kesselspeisewassers auf 150° C abgesenkt, kann auf die gleiche Weise eine Leistungssteigerung von 44 % bestimmt werden. Die so erreichbaren Steigerungen können entweder bei der Neuplanung von Anlagen genutzt werden, Reaktoren wirtschaftlicher auszulegen, oder um bestehende Anlagen zu ertüchtigen.

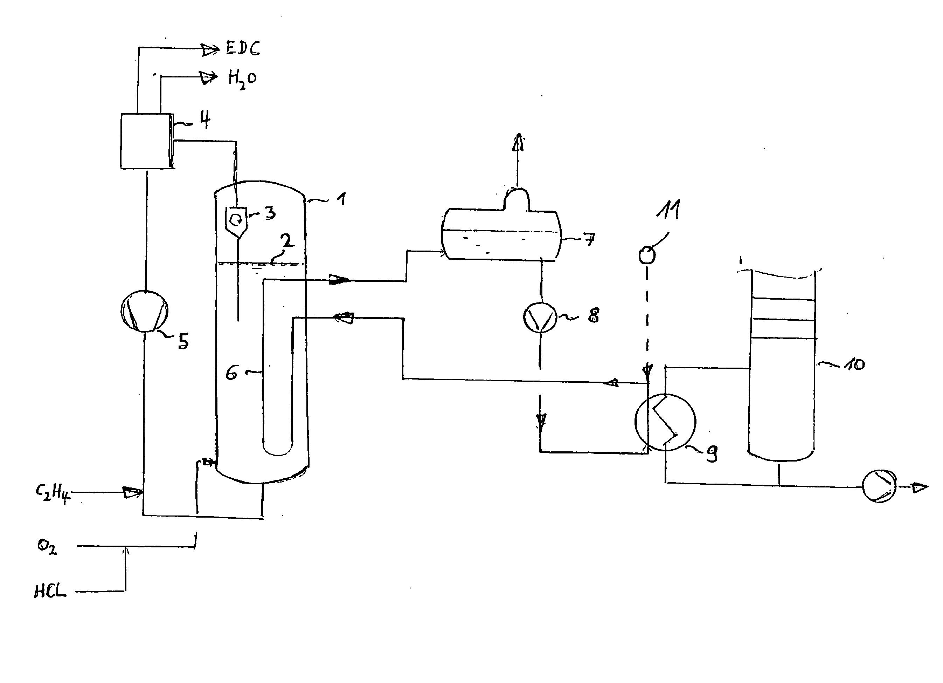

Die Erfindung ist nachstehend anhand der Zeichnung beispielsweise näher erläutert. Diese zeigt in vereinfachter Darstellung eine das erfindungsgemäße Verfahren nutzende Anlage im Anlagenschaltbild.

In dem mit 1 bezeichneten Oxichlorierungsreaktor befindet sich eine Wirbelschicht 2, aus der über einen Reaktorzyklon 3 bzw. eine entsprechende Katalysatorabtrennung das Produkt der Produktabtrennung 4 zugeführt wird.

Mit 5 ist dabei ein Kreisgasverdichter bezeichnet, der das Kreisgas in den Oxichlorierungsreaktor zurückführt, wobei die entsprechenden Edukte diesem Reaktor ebenfalls zugeführt werden.

Wesentlich für die Erfindung ist die Beaufschlagung des mit 6 bezeichneten Kühlrohrbündels mit abgekühltem Kesselspeisewasser aus einem Verdampfungsgefäß 7, wobei das Kesselspeisewasser über die Pumpe 8 umgewälzt wird.

Im dargestellten Beispiel - ohne dass die Erfindung hierauf beschränkt wäre - gibt das Kesselspeisewasser seine Wärme an einen Umlaufverdampfer 9 einer Destillationskolonne 10 ab und wird damit in der erfindungsgemäßen Weise abgekühlt. Mit 11 ist noch die Zuspeisung von Kesselspeisewasser bezeichnet.

Wie weiter oben schon erwähnt, ist die Erfindung nicht auf dieses Beispiel beschränkt. Über das Kesselspeisewasser können auch andere Anlagenelemente beheizt werden.

## Patentansprüche

1. Verfahren zur Nutzung der bei der Herstellung von 1,2-Dichlorethan aus Ethylen durch Umsetzung mit Sauerstoff und Chlorwasserstoff (Oxichlorierung) in einem Wirbelschichtreaktor entstehenden Reaktionswärme, mit Abführung dieser Reaktionswärme durch innerhalb des Reaktors befindliche in der Wirbelschicht positionierte Kühlrohrbündel,
**dadurch gekennzeichnet,**
**dass** ein Teil der Reaktionswärme durch Erwärmung von Kesselspeisewasser abgeführt wird, wobei das erwärmte Kesselspeisewasser zur Beheizung von Wärmesenken im Herstellungsprozess eingesetzt wird.

2. Verfahren nach Anspruch 1,
**dadurch gekennzeichnet,**
**dass** durch das durch die Reaktionswärme erwärmte Kesselspeisewasser Destillationskolonnen im Anlageverbund der Herstellung von 1,2-Dichlorethan/Vinylchlorid beheizt werden.

3. Verfahren nach Anspruch 1 oder 2,
**dadurch gekennzeichnet,**
**dass** durch das erwärmte Kesselspeisewasser Wärmetauscher zur Erwärmung von Prozessströmen im Anlagenverbund der Herstellung von 1,2-Dichlorethan/Vinylchlorid beheizt werden.

4. Verfahren nach einem der vorangehenden Ansprüche,
**dadurch gekennzeichnet,**
**dass** durch das erwärmte Kesselspeisewasser eine Trocknungsvorrichtung von Polyvinylchlorid (PVC) beheizt wird.

## Claims

1. Method for utilising the reaction heat which occurs in a fluidised bed reactor by means of reaction with oxygen and hydrogen chloride (oxychlorination) during the production of 1,2-dichloroethane from ethylene, together with dissipation of said reaction heat through cooling pipe bundles situated within the reactor and positioned in the fluidised bed, **characterised in that** a part of the reaction heat is dissipated by heating boiler feed water, the heated boiler feed water is being to heat heat sinks in the production process.

2. Method according to claim 1, **characterised in that** distillation columns in the facility network for the production of 1,2-dichloroethane/vinyl chloride are heated by the boiler feed water which is heated by the reaction heat.

3. Method according to either claim 1 or claim 2, **characterised in that** heat exchangers for heating process streams in the facility network for the production of 1,2-dichloroethane/vinyl chloride are heated by the heated boiler feed water.

4. Method according to any of the preceding claims, **characterised in that** a drying device for polyvinyl chloride (PVC) is heated by the heated boiler feed water.

## Revendications

1. Procédé d'utilisation de la chaleur réactionnelle produite lors de la fabrication de 1,2-dichloroéthane à partir d'éthylène par réaction avec de l'oxygène et du gaz chlorhydrique (oxychloration) dans un réacteur à lit fluidisé, avec évacuation de cette chaleur réactionnelle par un faisceau de tubes de refroidissement positionnés dans le lit fluidisé et se trouvant à l'intérieur du réacteur,
**caractérisé en ce que**
une partie de la chaleur réactionnelle est évacuée par chauffage d'eau d'alimentation de chaudière, l'eau d'alimentation de chaudière chauffée étant utilisée pour chauffer les puits de chaleur dans le processus de fabrication.

2. Procédé selon la revendication 1,
**caractérisé en ce que**
l'eau d'alimentation de chaudière chauffée par la chaleur réactionnelle permet de chauffer des colonnes de distillation en liaison opérationnelle avec la fabrication de 1,2-dichloroéthane/chlorure de vinyle.

3. Procédé selon la revendication 1,
**caractérisé en ce que**
l'eau d'alimentation de chaudière chauffée permet de chauffer des échangeurs de chaleur pour chauffer des courants du procédé en liaison opérationnelle avec la fabrication de 1,2-dichloroéthane/chlorure de vinyle.

4. Procédé selon l'une quelconque des revendications précédentes,
**caractérisé en ce que**
l'eau d'alimentation de chaudière chauffée permet de chauffer un dispositif de séchage de poly(chlorure de vinyle (PVC).
